# EUROPEAN PATENT APPLICATION

(11) **EP 4 641 581 A1**
(43) Date of publication of application: **29.10.2025**
(21) Application number: 24172219.8
(22) Date of filing: 24.04.2024
(51) Int. Cl.: G16H 30/20, G16H 30/40, G16H 40/67, G16H 80/00, G16H 20/40, G16H 50/50

(54) **REMOTE ASSISTANCE OF A MEDICAL INTERVENTION**

(71) Applicant: Siemens Healthineers AG, 91301 Forchheim (DE)
(72) Inventor: Fahrig, Rebecca, 91096 Möhrendorf (DE)
(74) Representative: Siemens Healthineers Patent Attorneys

(57) **Abstract**

For remote assistance of a medical intervention, an input specifying a proposed medical device is received by a data processing system (5). Representation data for generating a visual representation (8) of the medical device is generated by the data processing system (5) depending on the input and transmitted to a remotely located display device (7). The visual representation (8) is displayed by the display device (7) depending on the representation data and a display size of the display device (7) such that the displayed visual representation (8) corresponds the proposed medical device in real size or a further visual representation (8) of a reference object and the visual representation (8) are displayed by the display device (7) true to scale.

## Description

The present invention is directed to a method for remote assistance of a medical intervention, to a system for carrying out such a method, and to a corresponding computer program product.

Medical interventions, for example interventions on vessels including, but not limited to, cardiovascular interventions or interventions on cerebral vessels, may be carried out by a first person and/or robot, also denoted as operator in the following, who is assisted by a remotely located second person, also denoted as remote expert in the following. This is sometimes also denoted as remote surgery or telesurgery. This has for example the advantage that a single remote expert can assist several interventions at the same time or shortly one after the other and does not have to be present at the location of the intervention.

For carrying out such interventions, it may be necessary for the operator to select a medical device from a plurality of medical devices or to modify a generic medical device. The operator may be instructed by the remote expert to do so via remote verbal communication or text-based communication or gestures delivered via video link. It has been found that it is sometimes difficult for the operator to correctly understand or implement the instructions as intended by the remote expert. As a consequence, the operator may select the wrong medical device or modify the generic medical device in a wrong way and thus the invention process may be delayed.

It is an objective of the present invention to improve the remote assistance of a medical intervention such that the risk that the operator selects the wrong medical device or modifies the generic medical device in a wrong way is reduced.

This objective is achieved by the respective subject matter of the independent claim. Further implementations and preferred embodiments are subject matter of the dependent claims.

The invention is based on the idea to display a visual representation of a proposed medical device, which is proposed, in particular, by the remote expert to the operator, in real size or together with a reference object true to scale at the side of the operator.

According to an aspect of the invention, a method for remote assistance of a medical intervention is provided. Therein, an input, in particular a user input, in particular a user input of a remote expert, specifying a proposed medical device is received by a data processing system. Representation data for generating a visual representation of the proposed medical device is generated by the data processing system depending on the input. The representation data is transmitted from the data processing system to a display device, which is located remotely from the data processing system. The visual representation is displayed by the display device depending on the representation data and depending on a display size of the display device such that the displayed visual representation corresponds to the proposed medical device in real size. Alternatively, a further visual representation of a reference object and the visual representation are both displayed by the display device true to scale.

It is noted that the method according to the invention does not contain steps for carrying out the medical intervention, in particular by using the selected or modified medical device.

The input may, for example, be received by the data processing system via a user input interface including, for example, a speech input interface and/or a text input interface, for example from the remote expert. The user input interface may also be part of the data processing system.

For example, a medical device may selected from a plurality of medical devices according to the displayed visual representation or a generic medical device may be modified according to the displayed visual representation. The selection or modification may be carried out manually, automatically or in part automatically.

The selection of the device or the modification of the device is, for example, carried out by an operator or partly by the operator. The operator and the display device are located at the same location and remotely from the data processing system and, in particular, the remote expert. The operator and the display device are located, in particular, in an intervention room in which the medical intervention shall be carried out. That the data processing system and the display device are located remotely from each other may, for example, be understood such that the user input interface and, for example, the remote expert are located outside of the intervention room. The user input interface and the intervention room may, for example, be located far away from each other in the sense that it is not possible for the remote expert and the operator to communicate with each other directly verbally or to see each other directly.

The display device may be part of a further data processing system or may be connected to the further data processing system. Transmitting the representation data from the data processing system to the display device may therefore be realized by transmitting the representation data from the data processing system to the further data processing system and the further data processing system may control the display device to display the visual representation. In case the display device possesses a respective interface, it may also be possible that the representation data is transmitted from the data processing system directly to the display device.

The display device may for example comprise a two-dimensional array of pixels for displaying the visual representation. The array of pixels may have a width of W pixels and a height of H pixels. Consequently, a total number of pixels of the array of pixels is WxH. The display size may for example be given by W and H or, in other words, the total number of pixels WxH and the aspect ratio of the array of pixels, and a pixel size of the individual pixels of the array. These data may be stored in a memory of the display device, a memory of the data processing system, and/or a memory of the further data processing system.

The display size is for example received by the further data processing system. The further data processing system may then adapt the representation data accordingly depending on the display size such that the displayed visual representation corresponds to the proposed medical device in real size. Alternatively, the display size may be received by the data processing system and the data processing system may generate the representation data depending on the display size such that the further data processing system can control the display device accordingly to display the proposed medical device in real size.

The transmission of the representation data from the data processing system to the display device and/or to the further data processing system may, for example, include a transmission via a local area network, LAN, a wide area network, WAN, the internet, an ethernet connection, a Wi-Fi connection, a Bluetooth connection, and/or a cellular network.

The display device may be any type of device that is capable of displaying the visual representation based on the transmitted representation data, such as a computer screen, a screen of a television, a smartphone, or a tablet computer, a head-up-display device, a head-mounted display device, augmented reality glasses, virtual reality glasses, and so forth.

Some but not all of these types of display devices may be able to display the visual representation such that it corresponds to the proposed medical device in real size, which can be understood such that, if the real physical medical device would be held next to the displayed visual representation, both would have the same size and shape and proportions. This may, for example, be possible with a screen as the display device. With other types of display devices, such as a head-up-display device or augmented reality glasses, for example, this may not be possible or feasible. With such devices, it is, however, possible to display the visual representation together with the further visual representation of the reference object, which may be an anatomic object or a further medical device, true to scale, for example next to each other or one of them overlayed over the other, et cetera. Displaying the visual representation together with the further visual representation true to scale is, however, also possible with a screen as the display device.

The representation data may be any data, which is generated based on the input, which allows the display device to directly display the visual representation and, in some implementations, the further visual representation or which allows the further data processing system to control the display device to display the visual representation and, in some implementations, the further visual representation.

The medical device may be any medical device to be used during the medical intervention including, but not limited to, tools, implants and auxiliary devices. In particular, the medical device may be a catheter, in particular a vessel catheter, a stent, a vessel prosthesis or a guidewire for guiding a further medical device, such as a catheter or stent, in a vessel structure.

By means of the method according to the invention, the remote assistance of the operator by the remote expert to carry out the medical intervention is improved, since the risk that the operator selects the wrong medical device or modifies the generic medical device in a wrong way is reduced. This is achieved, in particular, by displaying the visual representation in real size or true to scale with respect to the reference object. Thus, the operator can directly verify their selection of the medical device or modification of the generic medical device based on the displayed visual representation.

In the present disclosure, the expressions "data processing system" and "at least one data processing device" may be used interchangeably. A data processing device may in particular be understood as a data processing device, which comprises processing circuitry. The data processing device can therefore in particular process data to perform computing operations. This may also include operations to perform indexed accesses to a data structure, for example a look-up table, LUT, as well as a data processing process implemented in hardware.

In particular, the data processing device may include one or more computers, one or more microcontrollers, and/or one or more integrated circuits, for example, one or more application-specific integrated circuits, ASIC, one or more field-programmable gate arrays, FPGA, and/or one or more systems on a chip, SoC. The data processing device may also include one or more processors, for example one or more microprocessors, one or more central processing units, CPU, one or more graphics processing units, GPU, and/or one or more signal processors, in particular one or more digital signal processors, DSP. The data processing device may also include a physical or a virtual cluster of computers or other of said units.

In various embodiments, the data processing device includes one or more hardware and/or software interfaces and/or one or more memory units.

A memory unit may be implemented as a volatile data memory, for example a dynamic random access memory, DRAM, or a static random access memory, SRAM, or as a non-volatile data memory, for example a read-only memory, ROM, a programmable read-only memory, PROM, an erasable programmable read-only memory, EPROM, an electrically erasable programmable read-only memory, EEPROM, a flash memory or flash EEPROM, a ferroelectric random access memory, FRAM, a magnetoresistive random access memory, MRAM, or a phase-change random access memory, PCRAM.

According to several implementations, a size, in particular a geometrical size, and/or a shape property, in particular a property of a geometrical shape, of the proposed medical device is displayed in real size or true to scale, in particular true to scale with respect to the reference object, by displaying the visual representation. The size and/or shape property may, for example, be specified by the input.

Consequently, the visual representation of the medical device provides an accurate portrayal of its dimensions and shape, allowing to accurately assess the proposed medical device's size and shape before making the selection or modification. In such implementations, an added level of precision and accuracy in the selection or modification of the medical device may be achieved. Displaying the size or shape in real size or true to scale can help to clarify uncertainties regarding the medical device, making the selection or modification process more efficient.

The size of the proposed medical device may correspond to dimensions of the proposed medical device or a specific part of it, which may for example be expressed in terms of length, width, height, diameter, thickness and so forth. In other words, the size can be understood as a measurable characteristic of the proposed medical device or said specific part of it, representing the extent or magnitude of its dimensions.

The shape property of the proposed medical device may refer to a characteristic of the proposed medical device that defines its geometric shape or form. This may include, for example, properties such as curvature, angles, contour and/or other geometric features. In case the proposed medical device is or includes a guide wire, the shape property may, for example, correspond to a curvature of a curved tip or an angle of a tilted tip of the guide wire et cetera.

According to several implementations, a medical device is selected from a plurality of medical devices according to the displayed visual representation or a generic medical device is modified according to the displayed visual representation. Sensor data representing the selected or modified medical device is generated by a sensor system. The selection or modification of the medical device is validated depending on the sensor data.

The sensor data may, for example, include information about the size, shape, or other physical characteristics of the selected or modified medical device, which may, for example, be compared to the proposed medical device. The validation of the selection or modification of the medical device depending on the sensor data may, for example, include confirming or rejecting the selection or modification of the medical device. If it is confirmed, then the operator may proceed using the selected or modified medical device and otherwise, another selection and/or modification may be required. This adds an additional level of precision and accuracy to the remote assistance method.

The sensor system may, for example, include one or more imaging sensors, such as a camera, a stereo camera, a 3D camera, for example a time-of-flight camera, and so forth. These may, for example, also be used to capture images of the selected or modified medical device from different angles. The sensor system may, for example, include one or more other optical sensors, such as laser scanners or optical probes, which may measure the dimensions and shape of the selected or modified medical device with particularly high precision. The sensor system may also include a touch-sensitive device, which may directly interact with the selected or modified medical device by touching the touch-sensitive device with the selected or modified medical device ("device-to-touch").

According to several implementations, the sensor data is transmitted to the data processing system, in particular by the sensor system. A validation output, in particular for the remote expert, is generated by the data processing system depending on the sensor data. The selection or modification of the medical device is validated in response to the validation output.

Consequently, it can be ensured with increased reliability that the selected or modified medical device is appropriate for the medical intervention. For example, the validation output may be a visual and/or auditory output that allows the remote expert to decide whether the selected or modified medical device is correctly selected or modified or not. The validation output may, for example, include the sensor data or a visual and/or auditory representation of the sensor data or a part of it. That the selection or modification of the medical device is validated in response to the validation output implies, in particular, that the validation is done after the validation output has been generated.

According to several implementations, a further input confirming or rejecting the selection or modification of the medical device is received by the data processing system, for example via the user input interface, in particular from the remote expert, in response to the validation output. A confirmation message is transmitted from the data processing system to the display device or to a further output device if the further input confirms the selection or modification of the medical device. The further output device is, in particular, located remotely from the data processing system. The further output device may, for example, be located in the intervention room.

If the confirmation message is received, for example by the operator, the operator may proceed further with using the selected or modified medical device for the medical intervention.

On the other hand, if the further input does not confirm, in particular does reject, the selection or modification of the medical device, a rejection message may be transmitted from the data processing system to the display device or to the further output device. The operator may then, for example, select another medical device or adapt the modification of the generic medical device or the steps of the method may be repeated to find the correct proposed medical device.

The further input confirming or rejecting the selection or modification may, in some implementations also be generated automatically, for example by an Al-based algorithms. The confirmation message or the rejection message may also be transmitted to the remote expert. The remote expert may also be prompted to suggest alternatives or changes and so forth.

According to several implementations, for generating the sensor data, the size and/or the shape property of the selected or modified medical device is measured by the sensor system. The selection or modification of the medical device is validated depending on the measured size and/or the shape property. In particular, the sensor data comprises the measured size and/or the shape property.

Consequently, a particularly reliable validation loop is established. The measurement may, for example, be carried out by the further data processing system and/or by a data processing device of the sensor system. For example, in case the sensor system is a laser scanner or optical probe, the sensor data may already include the respective measurement data. In case the sensor system is a camera, the measurement may include a computer program for measuring the size and/or the shape property being applied to the respective image or images. The computer program may, for example, include an object detection algorithm, an image segmentation algorithm, et cetera.

According to several implementations, for generating the sensor data, a camera image or a video of the selected or modified medical device is generated by at least one camera, in particular by at least one camera of the sensor system. The selection or modification of the medical device is validated depending on the camera image or the video. In particular, the sensor data comprises the camera image or the video.

According to several implementations, for generating the sensor data, a three-dimensional scan of the selected or modified medical device is generated by a laser scanner, in particular a laser scanner of the sensor system. The selection or modification of the medical device is validated depending on the three-dimensional scan.

This allows for a particularly precise characterization of the selected or modified medical device and thus for a particularly reliable validation. In particular, the sensor data comprises the three-dimensional scan.

According to several implementations, further sensor data representing the proposed medical device is generated by a further sensor system and the input comprises the further sensor data or is generated based on the further sensor data.

In other words, the user input is generated based on a real physical version of the proposed medical device. Consequently, the reliability of the process is further improved. For example, the remote expert may have the proposed medical device at hand and use the further sensor system to generate the input. The explanations and examples above regarding the sensor system apply analogously to the further sensor system.

According to a further aspect of the invention, a system for carrying out a method for remote assistance of a medical intervention according to the invention is provided. The system comprises the data processing system and the display device.

In some implementations, the system also comprises the sensor system and/or the further sensor system.

According to several implementations, the system comprises a robotic manipulator, which is configured to select the medical device from the plurality of medical devices according to the displayed visual representation and/or to modify the generic medical device according to the displayed visual representation.

The robotic manipulator may select the medical device or modify the generic medical device automatically, for example autonomously, or may be controlled by a human operator to do so.

Above and in the following, the solution according to the invention is described with respect to the claimed systems as well as with respect to the claimed methods according to the invention. Features, advantages, or alternative embodiments herein can be assigned to the other claimed objects and vice versa. In other words, claims and embodiments for the systems can be improved with features described or claimed in the context of the respective methods. In this case, the functional features of the method are implemented by physical units of the system.

According to a further aspect of the invention, a computer program comprising instructions is provided. When the instructions are executed by a system according to the invention, in particular by its data processing system, the instructions cause the data processing system to receive the input, generate the representation data, and transmit the representation data to the display device, and cause the display device to display the visual representation.

The instructions may be provided as program code, for example. The program code can, for example, be provided as binary code or assembler and/or as source code of a programming language, for example C, and/or as program script, for example Python.

According to several implementations, the instructions, when executed by the system according to the invention, in particular by its data processing system, cause the sensor system to generate sensor data and/or cause the sensor system to transmit the sensor data to the data processing system, and/or cause the data processing system to generate a validation output depending on the sensor data.

Further implementations of the computer program according to the invention follow directly from the various embodiments of the method according to the invention and vice versa. In particular, individual features and corresponding explanations as well as advantages relating to the various implementations of the method according to the invention can be transferred analogously to corresponding implementations of the computer program according to the invention.

According to a further aspect of the invention, a computer-readable storage medium storing a computer program according to the invention is provided.

The computer program and the computer-readable storage medium are respective computer program products comprising the instructions.

Further features and feature combinations of the invention are obtained from the figures and their description as well as the claims. In particular, further implementations of the invention may not necessarily contain all features of one of the claims. Further implementations of the invention may comprise features or combinations of features, which are not recited in the claims.

In the following, the invention will be explained in detail with reference to specific exemplary implementations and respective schematic drawings. In the drawings, identical or functionally identical elements may be denoted by the same reference signs. The description of identical or functionally identical elements is not necessarily repeated with respect to different figures.

In the figures,
- FIG 1: shows a schematic representation of an exemplary implementation of a system according to the invention;
- FIG 2: shows a schematic representation of a further exemplary implementation of a system according to the invention;
- FIG 3: shows a flow diagram of an exemplary implementation of a method for remote assistance of a medical intervention according to the invention;
- FIG 4: shows a flow diagram of a further exemplary implementation of a method for remote assistance of a medical intervention according to the invention; and
- FIG 5: shows a flow diagram of a part of a further exemplary implementation of a method for remote assistance of a medical intervention according to the invention.

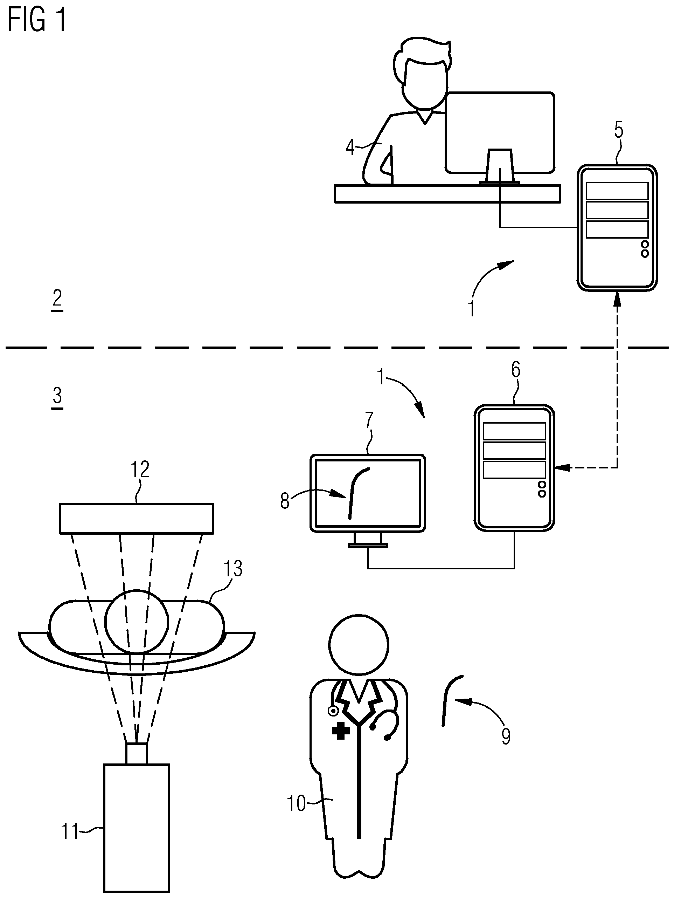

FIG 1 shows a schematic representation of an exemplary implementation of a system 1 for remote assistance of a medical intervention according to the invention.

The system 1 comprises a data processing system 5 for use by a remote expert 4. The system 1 further comprises a display device 7, which is located remotely from the data processing system 5 and the remote expert 4. The display device 7 is coupled to the data processing system 5, for example via a further data processing system 6, which is also located remotely to the data processing system 5. The display device 7 may display information to an operator 10, who may carry out the medical intervention. In particular, the remote expert 4 may communicate with the operator 10 via the data processing system 5, optionally the further data processing system 6, and the display device 7. In this way, the remote expert 4 may assist and/or instruct the operator 10 when carrying out the medical intervention.

The operator 10 and the display device 7 are located at the same location and remotely from the data processing system 5 and, in particular, the remote expert 4. The operator 10 and the display device 7 are, for example, located in an intervention room 3 in which the medical intervention shall be carried out. The data processing system 5 and the remote expert 4 are located outside of the intervention room, for example in a control room 2.

In a non-limiting example, the medical intervention may, for example, be a minimally-invasive intervention concerning a vessel structure of a patient 13 with fluoroscopic assistance by using an x-ray imaging system including an x-ray source 11 and an x-ray detector 12.

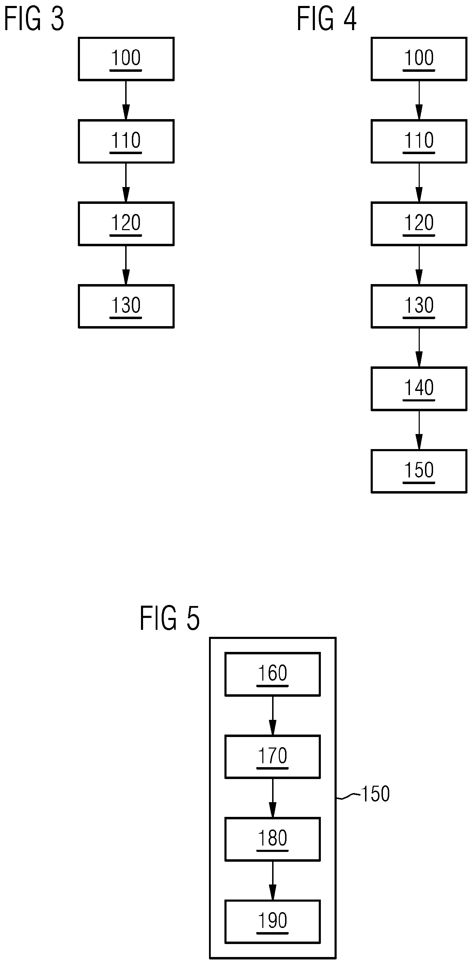

The system 1 is adapted to carry out a method for remote assistance of a medical intervention according to the invention. A corresponding flow diagram is shown in FIG 3.

In step 100, the data processing system 5 receives an input specifying a proposed medical device, in particular from the remote expert 4 via a user input interface of the data processing system 5. In step 110, the data processing system 5 generates representation data for generating a visual representation 8 of the proposed medical device depending on the received input and transmits it to the display device 7, for example via the further data processing system 6. In step 120, the visual representation 8 is displayed by the display device 7 depending on the representation data and depending on a display size of the display device 7 such that the displayed visual representation 8 corresponds to the proposed medical device in real size or a further visual representation of a reference object and the visual representation 8 are displayed by the display device 7 true to scale.

In optional step 130, a medical device is selected from a plurality of medical devices according to the displayed visual representation 8 or a generic medical device is modified according to the displayed visual representation, for example by the operator 10.

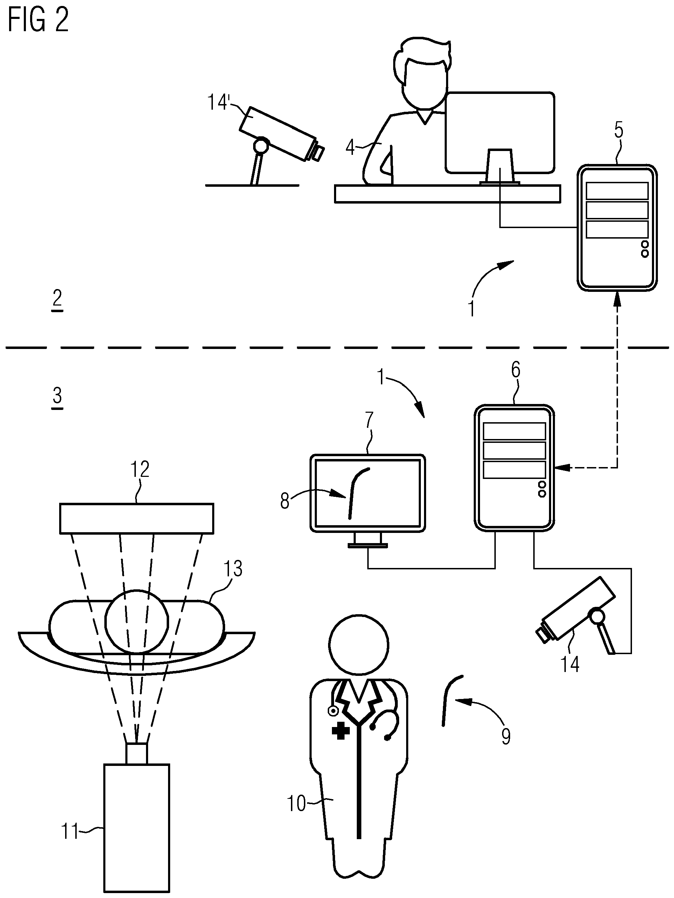

FIG 2 shows a schematic representation of a further exemplary implementation of a system 1 for remote assistance of a medical intervention according to the invention, which is based on the implementation of FIG 1.

Therein, the system 1 comprises a sensor system 14, such as a camera or a laser scanner or the like. The sensor system 14 may generate sensor data, for example one or more camera images or a video or a three-dimensional scan, and provide it, for example via the further data processing system 6 to the data processing system 5. FIG 3 shows a flow diagram of further exemplary implementation of a method for remote assistance of a medical intervention according to the invention, which may be carried out using the system 1 of FIG 2, and which is based on the method of FIG 3. Steps 100 to 130 of the method according to FIG 4 are identical to the steps 100 to 130 of the method according to FIG 3.

In step 140, sensor data representing the selected or modified medical device 9 is generated by the sensor system 14. In step 150, the selection or modification of the medical device is validated depending on the sensor data, for example by the remote expert 4.

FIG 5 shows a flow diagram of method steps of a further exemplary implementation of a method for remote assistance of a medical intervention according to the invention, which are comprised by step 150 of the method of FIG 4.

Therein, the sensor data is transmitted from the sensor system 140 to the data processing system 5, for example via the further data processing system 5, in step 160. In step 170, a validation output is generated by the data processing system 5 depending on the sensor data. In step 180, a further input confirming or rejecting the selection or modification of the medical device is received by the data processing system 5, in particular from the remote expert 4 via the user input interface of the data processing system 5, in response to the validation output. In step 190, a confirmation message is transmitted from the data processing system 5 to the display device 7 if the further input confirms the selection or modification of the medical device. The operator may then carry out the medical intervention using the selected or modified medical device 9.

As described, the invention improves the remote assistance of a medical intervention such that the risk that the operator 10 selects the wrong medical device or modifies the generic medical device in a wrong way is reduced.

For example in a vessel intervention, when the remote expert 4 picks the appropriate medical devices for the next step in the intervention procedure, they may determine that a particular shape or curvature needs to be added to the medical device in order to navigate through the vessels efficiently and effectively. The desired shape may be shown directly on the display device 7, which may for example be a computer screen, a touchpad screen or on a heads-up display, in real size or true to scale. Thus, a template is effectively provided to the operator 10, against which the medical device to be selected or modified can be compared directly.

In some implementations, direct feedback to the remote expert 4 may be provided via an optical camera, via device-to-touch screen interaction or via 3D scanning of the selected or modified medical device 9, in order to determine if the shape as provided by the operator 10 matches the proposed template. The direct feedback improves the remote expert's 4 confidence that the operator 10 has chosen the correct medical device and/or has implemented the correct shape modifications.

In some implementations, a 3D vision overlay may be used for the comparison of the proposed medical device against the true medical device.

In some implementations, an automated template selection may be provided. A prior imaging database that allows to determine, suggest and/or automatically calculate the best shape modification and/or device selection based on knowledge of a 3D vessel geometry of the patient 13 from prior 3D imaging technologies including, for example, computed tomography, magnetic resonance imaging, positron emission tomography, tomosynthesis, cone-beam computed tomography, and/or based on single-view angiography, two-view angiography, standard vessel geometries based on population statistics et cetera. Thus, proposed medical device or shape itself is provided automatically.

In some implementations remote expert 4 may have the exact medical devices in hand and/or modify a generic medical device, and then, for example through a further sensor device 14', such as a touch-display and/or an optical scanning device or a camera, the proposed medical device may be visualized to the operator 10.

Independent of the grammatical term usage, individuals with male, female or other gender identities are included within the term.

## Claims

1. Method for remote assistance of a medical intervention, wherein an input specifying a proposed medical device is received by a data processing system (5), representation data for generating a visual representation (8) of the proposed medical device is generated by the data processing system (5) depending on the input and is transmitted from the data processing system (5) to a display device (7), which is located remotely from the data processing system (5),
- the visual representation (8) is displayed by the display device (7) depending on the representation data and depending on a display size of the display device (7) such that the displayed visual representation (8) corresponds to the proposed medical device in real size; or
- a further visual representation of a reference object and the visual representation (8) are displayed by the display device (7) true to scale.

2. Method according to claim 1, wherein a size and/or a shape property of the proposed medical device is displayed in real size or true to scale by displaying the visual representation.

3. Method according to one of the preceding claims, wherein
- a medical device is selected from a plurality of medical devices according to the displayed visual representation (8) or a generic medical device is modified according to the displayed visual representation (8);
- sensor data representing the selected or modified medical device (9) is generated by a sensor system (14); and
- the selection or modification of the medical device is validated depending on the sensor data.

4. Method according to claim 3, wherein
- the sensor data is transmitted to the data processing system (5);
- a validation output is generated by the data processing system (5) depending on the sensor data; and
- the selection or modification of the medical device is validated in response to the validation output.

5. Method according to claim 4, wherein
- a further input confirming or rejecting the selection or modification of the medical device is received by the data processing system (5) in response to the validation output;
- a confirmation message is transmitted from the data processing system (5) to the display device (7) or to a further output device if the further input confirms the selection or modification of the medical device.

6. Method according to claim 2 and one of claims 3 to 5, wherein
- for generating the sensor data, the size and/or the shape property of the selected or modified medical device (9) is measured by the sensor system (14); and
- the selection or modification of the medical device is validated depending on the measured size and/or the shape property.

7. Method according to claim 2 and one of claims 3 to 5, wherein
- for generating the sensor data, a camera image or a video of the selected or modified medical device (9) is generated by at least one camera; and
- the selection or modification of the medical device is validated depending on the camera image or the video.

8. Method according to claim 2 and one of claims 3 to 5, wherein
- for generating the sensor data, a three-dimensional scan of the selected or modified medical device (9) is generated by a laser scanner; and
- the selection or modification of the medical device is validated depending on the three-dimensional scan.

9. Method according to one of the preceding claims, wherein further sensor data representing the proposed medical device is generated by a further sensor system and the input comprises the further sensor data or is generated based on the further sensor data.

10. Method according to one of the preceding claims, wherein the proposed medical device is a stent or a vessel prosthesis or a vessel catheter or a guidewire for guiding a further medical device in a vessel structure.

11. System (1) for carrying out a method according to one of the preceding claims, the system comprising the data processing system (5) and the display device (7).

12. System (1) according to claim 11 comprising a robotic manipulator, which is configured to select a medical device from a plurality of medical devices according to the displayed visual representation (8) or to modify a generic medical device according to the displayed visual representation (8).

13. Computer program product comprising instructions, which, when executed by a system (1) according to one of claims 11 or 12
- cause the data processing system (5) to receive the input, generate the representation data, and transmit the representation data to the display device (7); and
- cause the display device (7) to display the visual representation (8).
